Europäisches Patentamt

European Patent Office

Office européen des brevets

(11) Publication number: **0 400 762**
**A1**

# EUROPEAN PATENT APPLICATION

(21) Application number: **90201942.1**

(51) Int. Cl.⁵: **A61K 37/02**

(22) Date of filing: **19.09.86**

This application was filed on 16 - 07 - 1990 as a divisional application to the application mentioned under INID code 60.

(30) Priority: **20.09.85 US 778370**
**20.09.85 US 778371**
**20.09.85 US 778372**
**25.04.86 US 856035**
**25.04.86 US 856680**

(43) Date of publication of application:
**05.12.90 Bulletin 90/49**

(60) Publication number of the earlier application in accordance with Art.76 EPC: **0 219 979**

(84) Designated Contracting States:
**AT BE CH DE FR GB IT LI LU NL SE**

(71) Applicant: **CETUS CORPORATION**
**1400 Fifty-Third Street**
**Emeryville California 94608(US)**

(72) Inventor: **Nunberg, Jack Henry**
**5933 Chabot Road**
**Oakland, California 94618(US)**
Inventor: **Doyle, Michael Varian**
**2709 Mountaingate**
**Oakland, California 94611(US)**
Inventor: **Newell, Arthur Debney**
**45 Robert Road**
**Orinda, California 94563(US)**
Inventor: **White, Thomas James**
**5279 Clover Drive**
**Oakland, California 94618(US)**

(74) Representative: **Bizley, Richard Edward et al**
**HEPWORTH, LAWRENCE BRYER & BIZLEY**
**2nd Floor Gate House South West Gate**
**Harlow, Essex CM20 1JN(GB)**

(54) **Treating animals using IL-2, formulations therefor and their preparation.**

(57) Methods and formulations (and their preparation) are described for protecting an animal against stress-induced syndrome, which employ IL-2 as an active ingredient.

EP 0 400 762 A1

## TREATING ANIMALS USING IL-2, FORMULATIONS THEREOF AND THEIR PREPARATION

The invention is based upon a new use for interleukin-2 (IL-2). More particularly, it concerns using IL-2 in controlling stress-related diseases in animals.

Livestock food animals, particularly cattle, are adversely affected by shipment and feedlot conditions which involve stress from overcrowding, weaning, transport, sometimes severe weather, and, in general, a non-natural environment. One syndrome, commonly known as "shipping fever" is sometimes also designated "bovine respiratory disease syndrome" or BRDS. It is a complex of disease symptoms rather than a specific disease, and is characterized by immune suppression and propensity to succumb to infection by one or more viral or bacterial pathogens.

Other animals are also subject to adverse reactions to stress. For example, pigs, while ordinarily not shipped in the manner of cattle, can suffer negative respiratory reactions to weaning or just to poor weather. Again, the symptomatology does not lend itself to experimental models. No general treatment for stress-related disorders in livestock has been found. Sick animals are typically treated with antibiotics.

Recently several commercial entities have offered interferon preparations for treating shipping fever.

There is considerable background information available with respect to the biological activity of hIL-2. hIL-2 can be obtained from the supernatant of concanavalin-A (ConA) stimulated spleen cells or, presently, using recombinant technology, and has several measurable activities in vitro. First. it is a T-cell growth factor as measured by, for example, thymidine uptake when hIL-2 is added to cultures of cytotoxic or helper T-cell lines. It is mitogenic with respect to adult thymocytes, and stimulates a cytotoxic cell response (e.g., lymphokine-activated-killer (LAK) cell). It has also been shown to replace helper T-cells in athymic murine spleen cell cultures (Watson. J., et al. Immunological Rev (1980) 51:257-278). Specifically, in the presence of IL-2 and antigen, specific T helper cells are generated which are able to contribute to antibody responses. Presumably this occurs because hIL-2 is involved in the antigen-dependent maturation of helper T-cells in these nude mouse spleen cultures.

IL-2 has also been shown to directly affect B cells in vitro. Both proliferation and IgM and IgG secretion are enhanced by IL-2 in populations of purified, activated B cells (Mingari, M.C., et al., Nature (1984) 312:641; Mittler, R., et al., J. Immunol. (1985) 134: 2393-2399; Muraguchi, A., et al., J. Exp. Med. (1985) 161:181-197).

How these in vitro activities translate into a specific in vivo mechanism for mounting an immune defense is not clear. However. with respect to such in vitro studies, cross-reactivity among species of various IL-2s has been studied. For example, Redelman. D., et al, J Immunol Meth (1983) 56:359-370) show that hIL-2 supports activated T lymphocytes derived from rabbit and mouse to approximately the same extent as they are supported by the endogenous forms of IL-2. Ruscetti, F.W., et al, Blood (1981) 57:379-393 were the first to demonstrate the ability of hIL-2 to behave as a growth factor, not only for human T-cells, but also peripheral blood lymphocytes or splenocytes from other primates. horse, guinea pig, cat, rat, and mouse. Carter, J., et al (Fed Proc (1985) 44:1290) disclose the ability of hIL-2 to enhance the development and maintenance of bovine cytotoxic lymphocytes in vitro.

Doyle, M.V., et al, J Bio Resp Mod (1985) 4: 96-109 reports in vitro lymphocyte proliferation studies that compared the activities of native hIL-2 and a recombinant form of hIL-2 on human and animal lymphocytes. The native IL-2 and recombinant IL-2 exhibited the same range of activity on animal cells.

Some in vivo data are also available. The activity of IL-2 in vivo has been shown to restore immunocompetence in nude mice in response to heterologous erythrocytes (Stötter. H., et al, Eur J Immunol (1980) 10: 719-722). There is information concerning cross-species reactivity, as well. Reed, S.G., et al, J Immunol (1984) 133:3333, disclosed the ability of hIL-2 to reconstitute spleen cell responses in mice infected with a parasitic protozoan, and Farrar, J.J., et al, Immunol Rev (1982) 63:158, showed that in vivo injection of hIL-2 stimulates the splenic T-cells in nude mice.

In summary, it is known that hIL-2 behaves in some manner in vivo to mediate a successful immune response, including a response to a specific antigen, and in vitro studies have shown that cross-species reactivity of hIL-2 is very diverse (prior in vivo cross-species studies have involved only murine subjects for hIL-2).

However, because the nature of IL-2 activity in vivo is not completely understood and because the mechanism of involvement of IL-2 in the immune response is not understood, it is not, at this time, possible to predict its effect on particular diseases, other therapeutic or prophylactic regimens, or metabolism. Accordingly, there is no suggestion in the art that hIL-2 would successfully mitigate the incidence of stress-related syndromes in livestock.

One aspect of the invention is a formulation or composition for protecting an animal against stress-induced syndromes which comprises IL-2.

Another aspect is an IL-2 for use in protecting an animal against a stress-induced syndrome by a method which comprises administrating an effective amount of the IL-2 to the animal.

Also provided by the invention is the use of an IL-2 in preparing a medicament for protecting an animal against a stress-induced syndrome.

In a further aspect, the invention includes a process for preparing a formulation for protecting an animal against a stress-induced syndrome which process comprises formulating an IL-2 for such use.

Figure 1 shows the amino acid sequence of hIL-2.

Figure 2A and 2B are dose-response curves showing the results of the lypmphocyte proliferation tests described in section C.1 of the Examples, infra.

Figure 3 shows the effect of hIL-2 on blastogenesis of bovine and porcine T-lymphocytes.

## A. Definitions

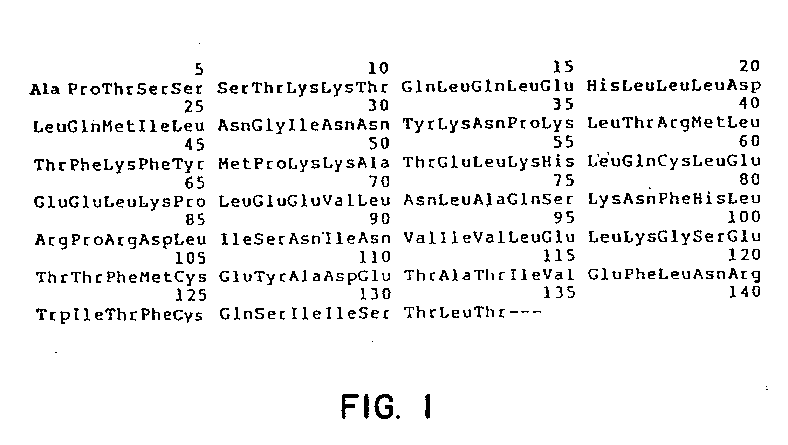

As used herein, "hIL-2" refers to a polypeptide exhibiting the spectrum of activities characterizing this protein. Specifically, the protein must be capable of stimulating the proliferation of hIL-2 dependent cytolytic and helper T cell lines, as set forth in the standard assays of Gillis, S, et al, J Immunol (1978) 120:2027-2032 and of Watson, J, J Exp Med (1979) 150: 1510-1519. The amino acid sequence of native hIL-2 is shown in Figure 1. This primary amino acid sequence may be obtained as the native protein from natural sources or may be recombinantly derived. Other primary sequences of modest modification including deletion, addition, substitution or alterations of the amino acids of the sequence shown, which do not result in serious impairment of activity are, of course, included in the definition. For example, it is established that replacement of the cysteine at position 125 with a neutral amino acid results in a mutein of superior stability and satisfactory reactivity. (See U.S. Patent No. 4,518,584; Doyle.,M.V., et al, supra.)

In addition, hIL-2, like any other protein, may exist in neutral or in salt form, and may contain associated nonprotein moieties in the nature of glycosylation, phosphorylation, or acetylation. These modifications, too, are included in the definition so long as biological activity is not destroyed thereby.

The word "IL-2" herein also includes bovine IL-2 as described by Cerretti et al., P.N.A.S. 83: 3223-3227 (1986)".

As used herein the term "stress-induced syndrome" refers to a state of immunosuppression in which an animal has a propensity to succumb to infection by one or more bacterial or viral pathogens, lose weight, or exhibit general ill health.

"Shipping fever" or "bovine respiratory disease syndrome" (BRDS) is defined as negative symptomatology including depression, immunosuppression, weight loss, respiratory problems, viral or bacterial infection, and general ill health and death which are associated with the transportation of cattle to, and the maintenance of cattle on, feedlots. The disease is defined in terms of epidemiology rather than in terms of a model which describes the course of an infection or specific set of metabolic parameters. The criterion for effectiveness against this disease is the maintenance of healthy animals faced with the specific conditions associated with shipping stress and feedlot maintenance. Certain parameters of the disease are recognized. It is characterized by an abrupt onset, usually within two weeks of stress, and the symptoms may include dyspnea, cough, ocular and nasal discharge, inappetance and rapid weight loss, fever, increased lung sounds, and general depression. Various bacteria and viral cultures have been isolated from affected animals, including Pasteurella spp, Haemophilus spp, infectious bovine rhinotracheitis, parainfluenzavirus, and bovine respiratory syncytial virus. The disease typically affects 40-50% of exposed animals and the resulting deaths are typically 2-5% of the exposed population.

## General Method

The formulations of the invention are most conveniently administered by intramuscular injections or as sustained release compositions although other methods of administration are possible. Specific formulations to prevent hydrolysis during digestion would be necessitated for oral formulation, and intravenous injections are generally uneconomic due to the skill level and care required in the administration. Therefore, formulations suitable for intramuscular injection, especially sustained release formulations, are preferred.

Standard formulations are either liquid injectables or solids which can be taken up in suitable liquids as suspensions or solutions for injection. Suitable excipients are, for example, water, saline, dextrose, glycerol, and ethanol. Nontoxic auxiliary substances, such as wetting agents, buffers, or emulsifiers may also be added. One specific useful formulation contains an effective amount of detergent, such as 0.1% sodium dodecyl sulfate (SDS), to effect solubility and bacteriostasis.

A variety of techniques are known in the art to effect long-term stability and slow release. For example, stability and half-life of hIL-2 are enhanced by coupling it to a hydrophilic polymer such as polyethylene glycol (PEG). This PEG-hIL-2 complex, called "PEGylated" hIL-2, is particularly useful for administering a single sustained action dose of hIL-2.

Sustained and continuous release formulations are of considerable variety, as is understood by those skilled in the art. An exemplary composition for sus tained release parenteral administration is an injectable microcapsule formulation that with a single injection will deliver recombinant hIL-2 or soluble forms of hIL-2, such as PEGylated hIL-2, at a controlled rate of about $10^3$ to $10^5$ units/kg/day (pure hIL-2 has a specific activity of about $3\text{-}6 \times 10^6$ u/mg). The micro-capsule formulation is a free-flowing powder consisting of spherical particles 20 to 100 $\mu$m in diameter that can be injected intramuscularly or subcutaneously with a conventional hypodermic needle, and the microcapsules consist of 0.5 to 5% hIL-2 encapsulated in poly-(DL-lactide-co-glycolide) (DL-PLG) excipient, a biodegradable, biocompatible polyester. Alternative standard formulations for sustained release are also usable.

The regime of hIL-2 administration for protecting animals against shipping fever will depend on the conditions of shipment and the feedlot. It is preferred that administration be continuous and be begun prior to shipment or at least as early as arrival on the feedlot and be continued over a period of, for example, 14-30 or more days. The term "continuous" is intended to denote true continuous administration, such as is achieved via a sustained release dosage form as well as a multiplicity of intermittent administrations of hIL-2 (or enhanced half-life forms of hIL-2 such as PEGylated hIL-2) that provide a pharmacokinetic pattern that mimics that achieved by true continuous administration. Daily doses in the range of above about $10^3$ and below about $10^6$ units/kg/day, preferably about $10^4$ to $10^5$ units/kg/day, are generally used. In cattle, doses above about $10^6$ units/kg/day began to cause undesirable side effects.

For other livestock stress-induced or respiratory distress syndromes, the regime and amounts administered will depend on the nature and size of the animal (e.g., pig, goat, sheep, etc.) and on the severity of the symptoms. It is likely, however, that the effective dose for such syndromes will be in the same (on a unit weight basis) range as that used for shipping fever.

The hIL-2 may be administered by itself or as a supplement to vaccines used to protect against stress-related diseases.

## C. Examples

The following examples are intended to further support or illustrate but not to limit the invention.

## C.1. In Vitro Activity

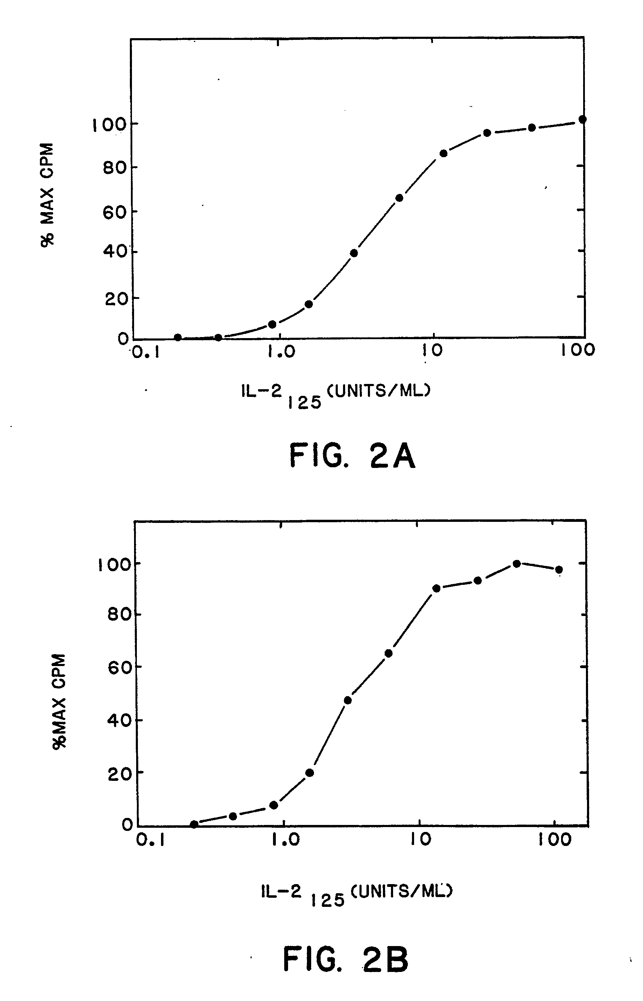

In vitro activity with respect to bovine and porcine peripheral blood mononuclear cells (PBMC) has been shown for recombinant hIL-2 (Fong, Susan, et al, Vet Immunol and Immunopathol (1986) 11:91-100). The hIL-2 used in this work is designated des-alanyl- rIL-2$_{ser125}$, lacks an initial alanine, and has a serine rather than a cysteine at position 125. It was shown to be mitogenic for unactivated bovine and porcine PBMC, and to be able to maintain the long-term growth of COnA-activated PBMC from both species. Figures 2A and 2B are curves showing the dose-response of ConA-activated bovine (2A) and porcine (2B) PBMC to des-alanyl-rIL-2$_{ser125}$. Also, bovine and porcine PBMC preincubated with des-alanyl-rIL-2$_{ser125}$ for 1-5 days showed enhanced cytotoxicity against tumor cell targets.

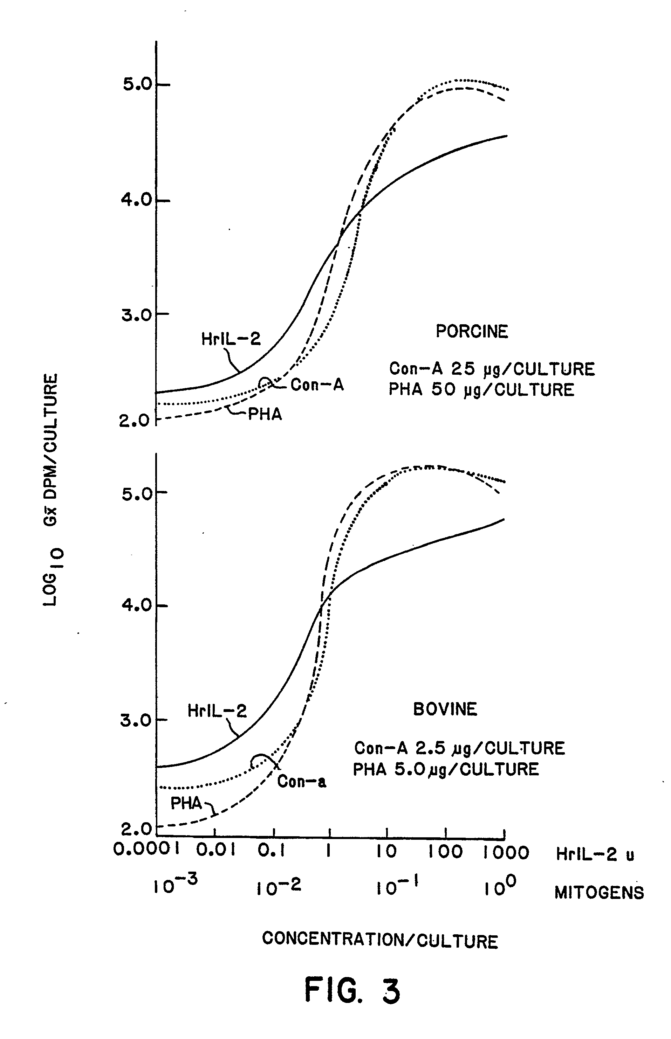

In addition, Stott, J.L., et al (in press) have shown that bovine and porcine peripheral blood lymphocytes were responsive to human recombinant IL-2 in lymphocyte blastogenesis assays. Blastogenesis was determined by incorporation of $^3$H-thymidine (18 hr pulse) in 4-day lymphocyte cultures, and the results expressed as the $\log_{10}$ of the geometric mean ($G_x$) of disintegrations per minute (DPM)/culture and plotted by nonlinear regression analysis as shown in Figure 3. Mitogen dilution and concentration of hIL-2 in units are shown on the X-axis. These results show that the effect of hIL-2 on bovine and porcine cells is comparable to that shown by the plant lectins PHA and ConA, which are known to stimulate blastogenesis.

## C.2. Potentiation of Cell-Mediated Immunity

Since respiratory diseases are predominantly controlled by the cellular (T-cell) immune system, the ability of hIL-2 to boost the cellular immune response in livestock is indicative of its effectiveness against these symptomologies. In vivo injections of recombinant hIL-2 produced elevated levels of lymphocyte blastogenesis in the blood of calves.

Specifically, eight calves weighing 135-225 kg (3-5 months old) were randomly sorted into 4 groups of 2 each which received weekly injections for one month as follows: Groups 1, 2, and 3 received $10^4$, $10^5$, and $10^6$ units/kg, respectively, intramuscularly; group 4 received only excipient. The animals were assessed for lymphocyte stimulation. The results show that resting lymphocyte activity was elevated by the recombinant hIL-2 treatment as determined by blastogenesis assays performed prior to each inoculation over the period in calves receiving $10^5$ and $10^6$ units/kg only. For calves receiving $10^5$ units/kg, lymphocyte activity returned to normal within two weeks following the last IL-2 administration; $10^6$ units/kg-injected calves remained elevated at that time.

## C.3. Treatment of Shipping Fever

Two hundred heifers were purchased from several different sources in Tennessee and transported to a research feedlot in Colorado. The average weight of the animals was approximately 180 kg. The animals were segregated randomly (weight and breed) into four groups, designated I through IV.

Recombinant hIL-2 (des-alanyl-rIL-2$_{ser125}$) was formulated in 0.05% SDS and administered intramuscularly to the animals upon entry to the feedlot. All animals were treated daily, five times per week, for two weeks. The dose protocols for the four groups were as follows.

| Group | IL-2 Dose (u/kg/day) |
|---|---|
| I | $2 \times 10^4$ (high dose) |
| II | $2 \times 10^3$ (mid dose) |
| III | $2 \times 10^2$ (low dose) |
| IV | control (diluent) |

The animals did not receive standard BRDS-related vaccination. They were, by chance, subjected to severe snow and cold weather during their first days on the feedlot, and accordingly, were placed on silage feed early on. The health of the animals was observed on a daily basis by personnel blind to experimental treatment. The animals were weighed at regular intervals. Table 1 reports the results of the treatment as of day 21.

Table 1

| Mortality | | |
|---|---|---|
| | Number Dead / Total | |
| Control<br>Low Dose<br>Mid Dose<br>High Dose | 21 / 50<br>20 / 50<br>26 / 50<br>14 / 50 | <br>p = 0.839<br>p = 0.316<br>p = 0.142 |
| Incidence of Disease | | |
| | Number Sick or Dead / Total | |
| Control<br>Low Dose<br>Mid Dose<br>High Dose | 43 / 50<br>42 / 50<br>32 / 50<br>38 / 50 | <br>p = 0.779<br>p = 1.000<br>p = 0.202 |
| Severity of Disease | | |
| | Average Daily Severity Score of Group (Score 0 - 3; Death = 4) | |
| Control<br>Low Dose<br>Mid Dose<br>High Dose | 1.76<br>1.79<br>1.93<br>1.38 | <br>p = 0.950<br>p = 0.395<br>p = 0.052 |

Morbidity and mortality rates during the study were higher than expected. As reported some groups showed 85% morbidity and 50% mortality. Sickness was observed as early as two days into the study. Several factors may have been responsible for the extreme severity of BRDS seen in this study: the severe snow and cold weather; the animals were 'light-weight' (400 lbs avg) and 'thin-skinned' (from Tennessee); groups had been 'put-together' from several sources (thus, they were not 'fresh' and many had seen several salebarns prior to shipping to Colorado); and the animals were placed on silage feed early on, and may have been eating poorly.

In the clinical judgement of the personnel observing the health of the animals, the high-dose IL-2 group consistently "looked better". This is supported by the data in Table 1 in which the high-dose IL-2 group showed a consistent trend towards decreased mortality; decreased incidence of disease; and decreased severity of disease.

In all cases, the high-dose group performed better than the control group. Although the statistical significance of these differences (p-value), is marginal (using the strict definition of $p < 0.05$), all results are consistent.

Additional measures not presented in Table 1 also supported the trend toward efficacy in the highdose IL-2 group. For instance, animals in the high-dose group which died, did so later in the study than did control animals.

As of day 21, there were no differences in the average weight of surviving animals. There were, however, significant differences in the total pay-weight per group, since more animals survived in the high-dose group.

## Claims

1. A formulation for protecting an animal against a stress-induced syndrome which formulation comprises an IL-2.

2. A formulation as claimed in Claim 1 wherein the IL-2 is hIL-2.

3. A formulation as claimed in Claim 2 wherein the hIL-2 is a water-soluble form of hIL-2.

4. A formulation as claimed in Claim 3 wherein the hIL-2 is PEGylated hIL-2.

5. A formulation as claimed in any one of Claims 2, 3, or 4 wherein the hIL-2 is des-alanyl-rIL-2 ser 125.

6. A formulation as claimed in any one of Claims 1 to wherein IL-2 is in the form of a continuous release formulation or is in the form of a single sustained action formulation.

7. An IL-2 for use in protecting an animal against a stress-induced syndrome by a method which comprises administrating an effective amount of the IL-2 to the animal.

8. The use of an IL-2 in preparing a medicament for protecting an animal against a stress-induced syndrome.

9. A process for preparing a formulation for protecting an animal against a stress-induced syndrome which process comprises formulating an IL-2 for such use.

10. An IL-2 as claimed in Claim 7, the use claimed in Claim 8, or a process as claimed in Claim 9, wherein the IL-2 is to be in the form of a continuous release formulation, the IL-2 being intended for administration continuously at a rate above $10^3$ and below $10^6$ units/kg/day, in each case optionally further defined by the specific feature of any one or more of Claims 2 to 5.

11. An IL-2 as claimed in Claim 7, the use claimed in Claim 8, or a process as claimed in Claim 9, in each case further defined by the specific feature of any one or more of Claims 2 to 6.

```
              5                  10                 15                 20
Ala ProThrSerSer SerThrLysLysThr GlnLeuGlnLeuGlu HisLeuLeuLeuAsp
             25                  30                 35                 40
LeuGlnMetIleLeu AsnGlyIleAsnAsn TyrLysAsnProLys LeuThrArgMetLeu
             45                  50                 55                 60
ThrPheLysPheTyr MetProLysLysAla ThrGluLeuLysHis LeuGlnCysLeuGlu
             65                  70                 75                 80
GluGluLeuLysPro LeuGluGluValLeu AsnLeuAlaGlnSer LysAsnPheHisLeu
             85                  90                 95                100
ArgProArgAspLeu IleSerAsnIleAsn ValIleValLeuGlu LeuLysGlySerGlu
            105                 110                115                120
ThrThrPheMetCys GluTyrAlaAspGlu ThrAlaThrIleVal GluPheLeuAsnArg
            125                 130                135                140
TrpIleThrPheCys GlnSerIleIleSer ThrLeuThr---
```

# FIG. I

EP 0 400 762 A1

FIG. 2A

FIG. 2B

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 534 594 (CETUS CORP.) * Page 28, lines 23-32; pages 30-33, claims * | 1-11 | A 61 K 37/02 |
| X,P | WO-A-8 504 328 (CETUS CORP.) * Pages 12-14 * | 1-11 | |
| A | WO-A-8 401 090 (ENDORPHIN INC.) * Pages 35-38, claims; page 32, lines 1-29; page 33, line 1 * | 1-11 | |
| A | EP-A-0 089 062 (AJINOMOTO CO.) * Pages 14-19; examples 1-18 * | 1-11 | |
| A | EP-A-0 154 316 (TAKEDA CHEMICAL INDUSTRIES) * Whole document * | 4,6,10 | |
| A | CHEMICAL ABSTRACTS, vol. 102, no. 7, 18th February 1985, page 444, abstract no. 60732g, Columbus, Ohio, US; G.T. SUKHIKH et al.: "Interleukin 2 and its possible role in the pathogenesis of stress-induced changes in the immune system", & DOKL. AKAD. NAUK SSSR 1984, 278(3), 762-5 [IMMUNOL.] * Abstract * | 1-11 | TECHNICAL FIELDS SEARCHED (Int. Cl.5)  A 61 K |

The present search report has been drawn up for all claims

| Place of search | Date of completion of the search | Examiner |
|---|---|---|
| THE HAGUE | 22-08-1990 | FERNANDEZ Y BRANAS F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)